# EUROPEAN PATENT SPECIFICATION

(11) **EP 0 912 471 B1**
(45) Date of publication and mention of the grant of the patent: **27.08.2003**
(21) Application number: 97931928.2
(22) Date of filing: 15.07.1997
(51) Int. Cl.: C07B 63/04, C07C 7/20

(54) **POLYMERISATION INHIBITOR**
POLYMERISATIONSINHIBITOR
INHIBITEUR DE POLYMERISATION

(30) Priority: 15.07.1996 GB 9614854
(43) Date of publication of application: 06.05.1999
(73) Proprietor: A.H. Marks & Co. Limited, Bradford West Yorkshire BD12 9EJ (GB)
(72) Inventor: PRYCE, Anthony, Leeds, West Yorkshire LS28 8BY (GB); CALLIN, Robert Charles, Ben Rhydding, Ilkley LS29 8QU (GB)
(74) Representative: Browne, Robin Forsythe, Dr.
(86) International application number: GB9701908
(87) International publication number: WO98002400

(56) References cited:
- EP-A- 0 550 754
- EP-A- 0 697 386
- WO-A-95/03263
- US-A- 5 254 760

## Description

This invention relates to free radical scavengers for use as or in connection with inhibitors of olefinic polymerisation, particularly but not exclusively for inhibition of polymerisation of vinyl aromatic compounds.

Olefinic compounds such as butadiene, acrylic monomers, styrene and other vinyl aromatic compounds have a strong tendency to polymerise on storage or heating. High temperature techniques such as distillation are commonly used during separation and purification processes and the industrial production of these olefinic compounds.

Although free radical scavengers have been used to prevent olefinic polymerisation, there is a requirement for higher efficiency scavengers which may be used at lower concentrations or for scavengers which are more efficient at the same concentrations as are currently employed. Lower concentrations are economic, less toxic and are environmentally beneficial. More efficient compositions are economic, giving reduced wastage and/or prolongation of the active life of the olefin.

Various compounds and compositions have been used as free radical scavengers to prevent or reduce undesired polymerisation of olefinic compounds during high temperature processes. These inhibitors have given varying degrees of success. In a typical process an olefinic compound may be contacted with the inhibitor before distillation or other processing. However the amount of polymer formed during such processing may be substantially higher than desired, leading to economic loss. In other cases it may be possible to achieve economically desirable low levels of polymerisation but only by use of economically unacceptable quantities of the inhibitor. Sulphur has been widely employed as a polymerisation inhibitor but difficulties in handling and disposal have lead to replacement by non-sulphur inhibitors, referred to as NSIs. SU-A-819078 discloses use of 3,5-ditertiary butyl-4-oxy-N,N-dimethylbenzylamine in combination with a C₁₀-C₂₀ aliphatic carboxylic acid. However the concentrations of this particular NSI are sufficiently large (500 to 2200 ppm) that much of the NSI is not consumed in a typical process. This results in uneconomic quantities of residual NSI being present in the effluent or waste from the process. EP-A-550754 discloses a polymerisation inhibitor for vinyl compounds combining a reaction product of 4-(dimethylamino)methyl-2,6-bis(tert butyl)phenol with an inorganic or carboxylic acid in the weight ratio 1 to 15:3. WO95/0326 discloses reduction in the quantity of this NSI by use in combination with air. However it is undesirable to introduce air into many high temperature processes involving olefinic compounds. Firstly there is an economic cost associated with ensuring a supply of the required quantity of air. Secondly it is often a safety hazard to introduce air into the stream of a hot olefinic compound due to the risk of oxidation, fire or an explosion.

Use of stable free radicals for inhibiting polymerisation by scavenging other free radicals has been disclosed. G M Burnett, Mechanism of Polymer Reactions, Interscience, New York 1954, 76, stated that a sterically hindered nitroxyl radical, 2,2,6,6-tetramethyl piperidinyl-1-oxyl (referred to as TEMPO) is effective against carbon centred radicals. Use of stable free radicals as scavengers was reviewed by E G Rozantsev, Free Nitroxyl Radicals, Plenan Press, 1970, 105. The practical utility of nitroxyl radicals as scavengers to prevent polymerisation was disclosed in GB 1127127 and GB 1218456. The high cost of stable free radicals has restricted their commercial use and there remains a requirement for an economic means of preventing or controling polymerisation. US 4670131 disclosed that certain stable free radicals may be used as polymerisation inhibitors under certain conditions at levels less than 700 parts per billion. However such low usage rates are not of general applicability.

We have now found that a free radical scavenger composition including an aromatic amine in combination with an organic acid and a stable free radical compound is effective without the economic disadvantages of the previously disclosed compositions.

According to a first aspect of the present invention a monomer composition, stabilised against premature polymerisation, comprises:
a) an ethylenically unsaturated monomer or mixture of monomers polymerisable by free radical initiation, and
b) an effective amount, sufficient to inhibit premature polymerisation of component (a) of a mixture of:
   i) 1 to 99% by weight, based on the total weight of components (i) and (ii) of a mixture of at least one aromatic amine and at least one organic acid in a molar ratio of 10:1 to 1:10, and
   ii) 99 to 1% by weight based on the total weight of components (i) and (ii) of at least one stable radical compound, wherein the aromatic amine has the formula (1) 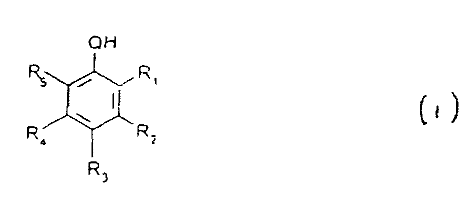 wherein Q is O or S or N-Z
      and wherein at least one R is an alkyl amine of the structure (2) 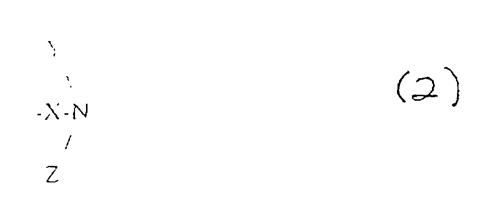 in which Y, Z are the same or different and comprise C₁ to C₄ branched or straight chain alkyl or hydrogen and in which X is either a covalent bond or C₁ to C₄ alkylene, and wherein each remaining R is independently benzyl, C₁ to C₄ branched or straight chain alkyl or hydrogen with the provision that two or more R may be connected to form one or more rings.
      Q is preferably oxygen.
      X is preferably methylene.
      Y and Z are preferably methyl.
      R¹ and R⁵ are preferably tertiary butyl.

Compositions in accordance with this invention exhibit synergistic effect in that the inhibition efficiency is greater than that of the two components. Preferred monomers are vinyl aromatic compounds for example styrene, methyl styrene, vinyl toluene or divinyl benzene or vinyl aliphatic compounds including acrylonitrile, acrylic acid, methacrylic acid, acrylate esters, methacrylate esters, butadiene and butenes. The invention is particularly applicable in inhibition of polymerisation of styrene during distillation, purification or storage.

In preferred compositions the weight of component i) is 50 to 99% and the weight of component ii) is 50 to 1%. In particularly preferred embodiments of the invention the weight of component i) is 67 to 99% and the weight of component ii) is 33 to 1%.

Percentages and other amounts in this specification are by weight unless indicated otherwise. Percentages and other proportions are selected to total 100%.

The molar ratio of said at least one aromatic amine to said at least one organic acid is 3:1 to 1:3.

A preferred amine has Y and Z as methyl and R² and R⁴ as hydrogen.

Preferred amines include benzylamine and benzylamine derivatives, for example 3,5-tertiarybutyl-4-hydroxybenzylamine, anilines and phenylene diamines, for example
N,N-dimethyl-1,4-phenylenediamine
3,5-ditertiarybutyl-4-oxy, N,N-dimethylbenzylamine
3,5-ditertiarybutyl-4-oxy-N,N-diethylbenzylamine
N,N-dimethyl-2,6-ditertiarybutyl-4-amino phenol
1,4-phenylenediamine
N,N-dimethyl-4-aminothiophenol
N,N'-bis-(1,4 dimethylpentyl)-1,4-phenylenediamine

In preferred embodiments of the invention the organic acid is a carboxylic acid. C₆ to C₂₂, preferably C₆ to C₁₈ saturated or unsaturated carboxylic acids are particularly efficacious. These may be substituted with groups inert to attack by the nitroxyl radical. Linear or branched chain acids may be employed. Branch chain acids which are liquid at ambient temperatures are convenient to manipulate. Unsubstituted saturated acids are especially preferred.

The stable radical compound is preferably a sterically hindered nitroxyl of the formulae (3) 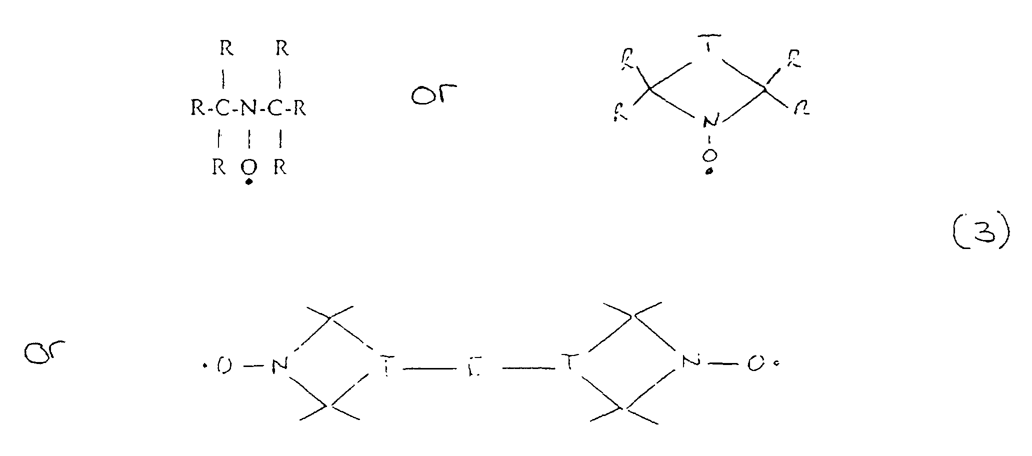 wherein R is hydrogen, alkyl or aryl and T is a group required to form a ring and wherein two or more T groups may be linked by a linking group E.

Preferred nitroxyl compounds may be selected from:
1-oxyl-2,2,6,6-tetramethylpiperidin,
1-oxyl-2,2,6,6-tetramethylpiperidin-4-ol,
1-oxyl-2,2,6,6-tetramethylpiperidin-4-one,
1-oxyl-2,2,6,6-tetramethylpiperidin-4-yl acetate,
1,oxyl-2,2,6,6-tetramethylpiperidin-4-yl 2-ethylhexanoate,
1-oxyl-2,2,6,6-tetramethylpiperidin-4-yl stearate,
1-oxyl-2,2,6,6-tetramethlpiperidin-4-yl benzoate,
1-oxyl-2,2,6,6-tetramethylpiperidin-4-yl 4-tert-butylbenzoate,
bis(1-oxyl-2,2,6,6-tetramethylpiperidin-4-yl)succinate,
bis(1-oxyl-2,2,6,6-tetramethylpiperidin-4-yl) sebacate,
bis(1-oxyl-2,2,6,6-tetramethylpiperidin-4-yl) sebacate,
bis(1-oxyl-2,2,6,6-tetramethylpiperidin-4-yl) adipate,
bis(1-oxyl-2,2,6,6-tetramethylpiperidin-4-yl) n-butylmalonate,
bis(1-oxyl-2,2,6,6-tetramethylpiperidin-4-yl)phthalate,
bis(1-oxyl-2,2,6,6-tetramethylpiperidin-4-yl) iscphthalate,
bis(1-oxyl-2,2,6,6-tetramethylpiperidin-4-yl)terephthalate,
bis(1-oxyl-2,2,6,6-tetramethylpiperidin-4-yl) hexahydoterephthalate,
N,N'bis(1-oxyl-2,2,6,6-tetramethyulpiperidin-4-yl) adipamine,
N-(1-oxyl-2,2,6,6-tetramethylpiperidin-4-yl) caprolactam,
N-(1-oxyl-2,2,6,6-tetramethylpiperidin-4-yl)-dodecylsuccinimide
2,4,6-tris-[N-butyl-N-(1-oxyl-2,2,6,6-tetramethylpiperidin-4-yl]-s-triazine, and
4,4'-ethylenebis(1-oxyl-2,2,6,6-tetramethylpiperidin-3-one)
4-acetylamino 2,2,6,6-tetramethylpiperidin-N-oxyl

Most preferably, the compound of component b) is
1-oxyl-2,2,6,6-tetramethylpiperidin-4-ol, or
bis(1-oxyl-2,2,6,6-tetramethylpiperidin-4-yl) sebacate

Preferred nitroxyl compounds are piperidinoxyl compounds.

According to a second aspect of the present invention a process for inhibiting premature polymerisation of a vinyl aromatic or aliphatic compound during distillation, purification or storage, comprises the step of incorporating therein an effective inhibiting amount, sufficient to prevent premature polymerisation during distillation, purification or storage of a mixture of
i) 1 to 99% by weight, based on the total weight of components (i) and (ii), of an admixture of at least one aromatic amine and at least on organic acid in a molar ratio of from 10:1 to 1:10 and
ii) 99 to 1% by weight, based on the total weight of components (i) and (ii), of at least one stable radical compound.

Preferred processes of this invention use a stabilising composition which is synergistic in nature, that is using half the concentration of component a) together with half the concentration of component b) to afford a greater stabilisation efficacy than may be achieved using either component a) or b) alone.

Preferred processes in accordance with this invention employ stabilising compositions as described above with reference to the first aspect of this invention.

The effective amount of polymerisation inhibitors employed may vary over a wide range depending upon the particular olefinic compound and the distillation or other purification conditions employed. Preferably the total amount of an aromatic amine and an aliphatic carboxylic acid and a stable free radical is from 1 ppm to 2000 ppm based upon the weight of the olefinic compound. For most olefinic compounds the mixture of components a) and b) is 5 to 1000 ppm. Greater amounts of inhibitor are required at higher temperatures. During distillation of olefinic mixtures the temperature of the reboiler may be up to about 150°C. Since the boiling points of the various aromatic amines of component a) and of the various stable free radicals b) are different, compounds which have the desired boiling points can be easily selected. The compositions of this invention which inhibit the polymerisation of the olefinic compound are also well suited for protecting the re-boiler sections of a distillation column or the compressor sections before entering a distillation column.

The polymerisation inhibitor can be introduced into the olefinic compound to be protected by any conventional method. It may be added as a concentrated solution in a suitable solvent. Since the solubilities of the various aromatic amines of component a) of the various organic acids of component a) and of the various stable free radicals of component b) are different, compounds which have the desired solubilities can be easily selected. The components a) and b) may be injected separately into the distillation train along with the incoming feed, or through separate entry points providing there is an efficient distribution of the inhibitors.

The inhibitors are gradually depleted during the distillation operation. Consequently it is generally necessary to maintain the appropriate amount of inhibitor mixture in the distillation apparatus by adding inhibitors during the course of the distillation process. Such addition may be carried out either on a generally continuous basis or it may consist of intermittently charging inhibitor into the distillation system in order to maintain the concentration of the inhibitor in excess of the minimum required level. The amine and carboxylic acid may be added separately at different entry points into the process stream.

The present invention enables the distillation and purification of olefinic compounds in manufacturing plants to operate more economically compared to prior art processes because of the greater effectiveness with or without the presence of oxygen. This permits lower inhibitor usages with minimal polymer formation.

The present invention also enables the storage of olefinic compounds for prolonged periods of time at ambient or elevated temperatures with or without the presence of oxygen by utilising economically beneficial amounts of the scavenger composition to minimise polymer formation.

The present invention is further described by means of example but not in any limitative sense.

### Examples 1 to 3

Commercial quality styrene was washed with dilute caustic soda solution to remove the storage stabiliser, tert-butyl-catechol. The styrene was then washed with water to remove excess alkali, following which it was dried over anhydrous sodium sulphate prior to use as follows;

Inhibitor and styrene (enough to make total weight of mixture of 200 g) were charged to a 250 ml round-bottomed flask fitted with an overhead stirrer, thermometer, sparge tube and water cooled condenser (see diagram below). The stirrer was started. Nitrogen (200 ml min⁻¹) sparging through the reaction mixture was then applied. The reaction mixture was then heated to 100°C using an oil bath, and stirred at this temperature for two hours. Samples were removed and analysed for polystyrene content using a spectrophotometric method at 420 nm. The analysis method was based on American Standard Test Method D2121 and a calibration was made using authentic polystyrene in styrene solutions of known concentrations by weight. In a controlled experiment, without any inhibitor present much greater than 30000 ppm (limit of quantification) of polystyrene was formed. Polymer levels obtained with various inhibitors present are shown in the table below. The blends of inhibitors were considerably more effective at reducing the amount of polymer formed than were either of the components wnen used individually at the full race of inhibitor concentration. This clearly demonstrates that an unexpected synergistic effect occurs in the polymerisation inhibition of styrene in the absence of air by using both an aromatic amine-aliphatic carboxylic acid inhibitor and a stable free radical inhibitor together as compared to using either inhibitor alone at twice its concentration found in the combination.

| **Example No** | **Inhibitors** | **ppm** | **Polymer (ppm)** |
|---|---|---|---|
| Control | None | --- | >30,000 |
| 1 | A | 33 | 23 448 |
| 2 | B | 517 | 754 |
| 3 | A plus B | 17 plus 258 | 50 |

A is 4-hydroxy-2,2,6,6-tetramethyl-piperidinyl-1-oxyl radical. B is 3,5-ditertiarybutyl-4-oxy, N,N-dimethylbenzylamine in combination with stearic acid (molar ratio 1:1.5).

### Examples 4 to 6

The procedure of Examples 1 to 3 was repeated with a higher test temperature of 115°C and instead of a nitrogen sparge there was applied an air sparge (4 ml min⁻¹). The results of these tests are shown below;

| **Example No** | **Inhibitors** | **ppm** | **Polymer (ppm)** |
|---|---|---|---|
| 4 | A | 33 | 28 000 |
| 5 | B | 517 | 3 000 |
| 6 | A plus B | 17 plus 258 | 1 684 |

A is 4-hydroxy-2,2,6,6-tetramethyl-piperidinyl-1-oxyl radical B is 3,5-ditertiary butyl-4-oxy-N,N-dimethylbenzylamine in combination with stearic acid (molar ratio 1:1.5).

There is a clear synergistic inhibiting effect on the polymerisation of styrene in the presence of air. When a mixture of the two inhibitor compounds are used together as compared to using either inhibitor alone at twice its concentration used in the combination.

### Examples 7 to 9

The procedure of Examples 1 to 3 was repeated and instead of nitrogen sparge there was applied an air sparge (4 ml min⁻¹).

| **Example No** | **Inhibitors** | **ppm** | **Polymer (ppm)** |
|---|---|---|---|
| 7 | A | 10 | 14 |
| 8 | C | 150 | 18,230 |
| 9 | A plus C | 5 plus 75 | 0 |

A is 4-hydroxy-2,2,6,6- tetramethyl-piperidinyl-1-oxy radical C is 3,5-ditertiary butyl-4-oxy N,N-dimethylbenzylamine in combination with stearic acid (molar ratio 1:3)

### Examples 10 to 12

The procedure of Examples 7 to 9 was repeated but the time at 100°C was 1 hour instead of 2 hours.

| **Example No** | **Inhibitors** | **ppm** | **Polymer (ppm)** |
|---|---|---|---|
| 10 | A | 2 | 1,562 |
| 11 | D | 30 | 1,684 |
| 12 | A plus D | 1 plus 15 | 49 |

A is 4-hydroxy-2,2,6,6-tetramethyl-piperidinyl-1-oxy radical D is 1,4-phenylenediamine in combination with stearic acid (molar ratio 1:1.5)

### Examples 13 to 15

The procedure of Examples 4 to 6 was repeated but the time at 115°C was 1 hour instead of 2 hours.

| **Example No** | **Inhibitors** | **ppm** | **Polymer (ppm)** |
|---|---|---|---|
| 13 | A | 10 | 23,274 |
| 14 | E | 150 | 101 |
| 15 | A plus E | 5 plus 75 | 0 |

A is 4-hydroxy-2,2,6,6-tetramethyl-piperidinyl-1-oxy radical E is N,N-bis-(1,4-dimethylpentyl)-1,4-phenylenediamine in combination with stearic acid (Molar Ratio 1:1.5)

### Examples 16 to 21

The procedure of Examples 1 to 3 were repeated but a molar equivalent quantity of capric/caprylic acid was used in place of the stearic acid without any significant difference in the results.

### Examples 22 to 24

The procedures of Examples 4 to 6 were repeated but a molar equivalent quantity of capric/caprylic acid was used in place of the stearic acid without any significant difference in the results.

### Examples 25 to 27

The procedures of Examples 7 to 9 were repeated.

| **Example No** | **Inhibitors** | **ppm** | **Polymer (ppm)** |
|---|---|---|---|
| 25 | F | 10 | 414 |
| 26 | B | 150 | 19,970 |
| 27 | F plus B | plus 75 | 397 |

B is 3,5-ditertiarybutyl 4 xy N,N-dimethylbenzylamine in combination with stearic acid (Molar Ratio 1:1.5) F is ditertiary butyl nitroxide.

## Claims

1. A monomer composition, stabilised against premature polymerisation, comprising:
a) an ethylenically unsaturated monomer or mixture of monomers polymerisable by free radical initiation, and
b) an effective amount, sufficient to inhibit premature polymerisation of component (a) of a mixture of:
i) 1 to 99% by weight, based on the total weight of components (i) and (ii) of a mixture of at least one aromatic amine and at least one organic acid in a molar ratio of 10:1 to 1:10, and
ii) 99 to 1% by weight based on the total weight of components (i) and (ii) of at least one stable radical compound,
wherein the aromatic amine has the formula (1) 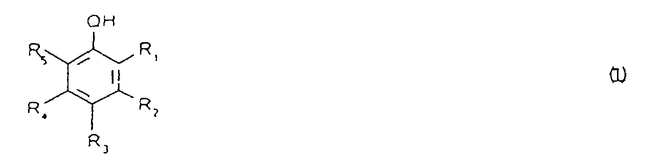 (wherein Q is O or S or N-Z
and wherein at least one R is an alkyl amine of the structure (2) 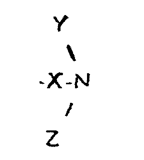 in which Y, Z are the same or different and comprise C₁ to C₉ branched or straight chain alkyl or hydrogen and in which X is either a covalent bond or C₁ to C₄ alkylene, and wherein each remaining R is independently benzyl, C₁ to C₄ branched or straight chain alkyl or hydrogen with the provision that two or more R may be connected to form one or more rings) ;
wherein the organic acid is a carboxylic acid; and
wherein the stable radical compound is a sterically hindered nitroxyl of formula 3 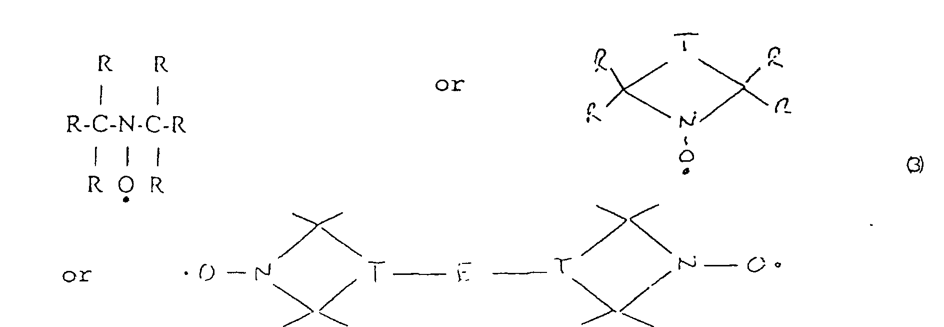 (wherein R is hydrogen, alkyl or aryl and T is a group required to form a ring and wherein two or more T groups may be linked by a linking group E).

2. A composition as claimed in claim 1, wherein the weight of component (i) is 50 to 99% and the weight of component (ii) is 50 to 1%.

3. A composition as claimed in claim 2, wherein the weight of component (i) is 67 to 99% and the weight of component (ii) is 33 to 1%.

4. A composition as claimed in any preceding claim, wherein the molar ratio of the aromatic amine to organic acid is 3:1 to 1:3.

5. A composition as claimed in claim 1, wherein Q is oxygen.

6. A composition as claimed in claim 1 or 5 wherein X is methylene.

7. A composition as claimed in any of claims 1, 5 or 6, wherein Y and Z are methyl.

8. A composition as claimed in any preceding claim, wherein the amine is selected from
N,N-dimethyl-1,4-phenylenediamine
3,5-ditertiarybutyl-4-oxy, N,N-dimethylbenzylamine
3,5-ditertiarybutyl-4-oxy-N,N-diethylbenzylamine
N,N-dimethyl-2,6-ditertiarybutyl-4-amino phenol
1,4-phenylenediamine
N,N-dimethyl-4-aminothiophenol
N,N'-bis-(1,4 dimethylpentyl)-1,4-phenylenediamine.

9. A composition as claimed in claim 1, wherein the organic acid is a C₆ to C₂₂ carboxylic acid.

10. A composition as claimed in claim 1, wherein the organic acid is a C₆ to C₁₈ carboxylic acid.

11. A composition as claimed in claim 1, wherein the nitroxyl compound is selected from:
1-oxyl-2,2,6,6-tetramethylpiperidine,
1-oxyl-2,2,6,6-tetramethylpiperidin-4-ol,
1-oxyl-2,2,6,6-tetramethylpiperidin-4-one,
1-oxyl-2,2,6,6-tetramethylpiperidin-4-yl acetate,
1,oxyl-2,2,6,6-tetramethylpiperidin-4-yl 2-ethylhexanoate,
1-oxyl-2,2,6,6-tetramethylpiperidin-4-yl stearate,
1-oxyl-2,2,6,6-tetramethlpiperidin-4-yl benzoate,
1-oxyl-2,2,6,6-tetramethylpiperidin-4-yl 4-tert-butylbenzoate,
bis(1-oxyl-2,2,6,6-tetramethylpiperidin-4-yl)succinate,
bis(1-oxyl-2,2,6,6-tetramethylpiperidin-4-yl) sebacate,
bis(1-oxyl-2,2,6,6-tetramethylpiperidin-4-yl) sebacate,
bis(1-oxyl-2,2,6,6-tetramethylpiperidin-4-yl) adipate,
bis(1-oxyl-2,2,6,6-tetramethylpiperidin-4-yl) n-butylmalonate,
bis(1-oxyl-2,2,6,6-tetramethylpiperidin-4-yl)phthalate,
bis(1-oxyl-2,2,6,6-tetramethylpiperidin-4-yl) isophthalate,
bis(1-oxyl-2,2,6,6-tetramethylpiperidin-4-yl)terephthalate,
bis(1-oxyl-2,2,6,6-tetramethylpiperidin-4-yl) hexahydoterephthalate,
N,N'bis(1-oxyl-2,2,6,6-tetramethyulpiperidin-4-yl) adipamine,
N-(1-oxyl-2,2,6,6-tetramethylpiperidin-4-yl) caprolactam,
N-(1-oxyl-2,2,6,6-tetramethylpiperidin-4-yl)-dodecylsuccinimide
2,4,6-tris-[N-butyl-N-(1-oxyl-2,2,6,6-tetramethylpiperidin-4-yl]-s-triazine, and
4,4'-ethylenebis(1-oxyl-2,2,6,6-tetramethylpiperidin-3-one)
4-acetylamino 2,2,6,6-tetramethylpiperidin-N-oxyl.

12. A composition as claimed in claim 1, wherein the nitroxyl is 1-oxyl-2,2,6,6-tetramethylpiperidin-4-ol, or bis(1-oxyl-2,2,6,6-tetramethylpiperidin-4-yl) sebacate.

13. A process for inhibiting premature polymerisation of a vinyl aromatic or aliphatic compound during distillation, purification or storage, comprising the step of incorporating therein an effective inhibiting amount, sufficient to prevent premature polymerisation during distillation, purification or storage of a mixture of
i) 1 to 99% by weight, based on the total weight of components (i) and (ii), of an admixture of at least one aromatic amine and at least on organic acid in a molar ratio of from 10:1 to 1:10 and
ii) 99 to 1% by weight, based on the total weight of components (i) and (ii), of at least one stable radical compound,
wherein said aromatic amine, said organic acid and said radical compound are as defined in claim 1.

14. A process as claimed in claim 13, wherein said mixture is selected to provide a composition in accordance with any of claims 1 to 12.

15. A process as claimed in claim 13 or 14.

## Patentansprüche

1. Monomerzusammensetzung, die gegen eine vorzeitige Polymerisation stabilisiert ist und umfasst:
a) ein (e) ethylenisch ungesättigte (s) Monomer oder Mischung aus Monomeren, das (die) durch freie radikalische Inhibierung polymerisierbar ist und
b) eine effektive Menge, die ausreicht, um die vorzeitige Polymerisation von Bestandteil (a) zu inhibieren, einer Mischung aus:
i) 1 bis 99 Gew.-%, bezogen auf das Gesamtgewicht der Bestandteile (i) und (ii), einer Mischung aus mindestens einem aromatischen Amin und mindestens einer organischen Säure in einem Molverhältnis von 10:1 bis 1:10 und
ii) 99 bis 1 Gew.-%, bezogen auf das Gesamtgewicht der Bestandteile (i) und (ii), mindestens einer stabilen radikalischen Verbindung,
worin das aromatische Armin die Formel (1) aufweist: 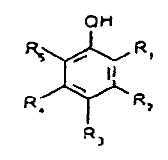 (worin Q O oder S oder N-Z bedeutet und worin mindestens ein R ein Alkylamin der Struktur(2)ist: 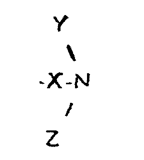 worin Y, Z gleich oder unterschiedlich sind und C₁-C₉-verzweigtes oder geradkettiges Alkyl oder Wasserstoff umfassen und worin X entweder eine kovalente Bindung oder C₁-C₄-Alkylen bedeutet und worin jedes verbleibende R voneinander unabhängig Benzyl, C₁-C₄-verzweigtes oder geradkettiges Alkyl oder Wasserstoff bedeutet, mit der Maßgabe, dass zwei oder mehrere von R verbunden sein können und einen oder mehrere Ringe bilden);
worin die organische Säure eine Carbonsäure bedeutet und worin die stabile radikalische Verbindung ein sterisch gehindertes Nitroxyl der Formel(3)bedeutet: 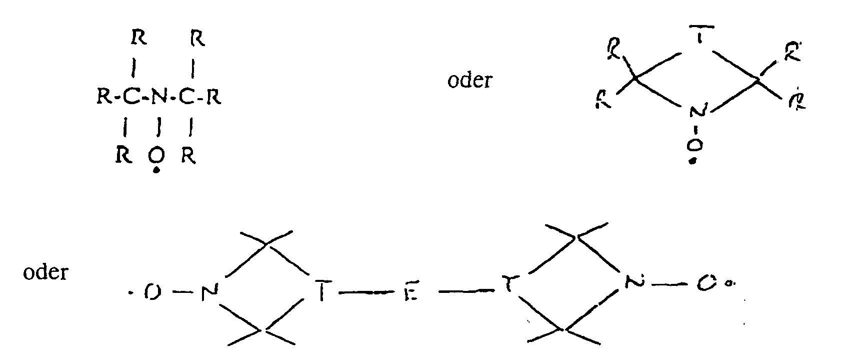 (worin R Wasserstoff, Alkyl oder Aryl bedeutet und T eine Gruppe bedeutet, die dafür erforderlich ist, einen Ring zu bilden, und worin zwei oder mehrere T-Gruppen über eine Verbindungsgruppe E verbunden sein können).

2. Zusammensetzung nach Anspruch 1, worin das Gewicht von Bestandteil (i) 50 bis 99 % beträgt und das Gewicht von Bestandteil (ii) 50 bis 1 % beträgt.

3. Zusammensetzung nach Anspruch 2, worin das Gewicht von Bestandteil (i) 67 bis 99 % beträgt und das Gewicht von Bestandteil (ii) 33 bis 1 % beträgt.

4. Zusammensetzung nach einem der vorangegangenen Ansprüche, worin das Molverhältnis des aromatischen Amins zur organischen Säure 3:1 - 1:3 beträgt.

5. Zusammensetzung nach Anspruch 1, worin Q Sauerstoff bedeutet.

6. Zusammensetzung nach Anspruch 1 oder 5, worin X Methylen bedeutet.

7. Zusammensetzung nach einem der Ansprüche 1, 5 oder 6, worin Y und Z Methyl bedeuten.

8. Zusammensetzung nach einem der vorangegangenen Ansprüche, worin das Amin aus
N,N-Dimethyl-1,4-phenylendiamin,
3,5-Di-tert.-butyl-4-oxy, N,N-Dimethylbenzylamin,
3,5-Di-tert.-butyl-4-oxy-N,N-diethylbenzylamin,
N,N-Dimethyl-2,6-di-tert.-butyl-4-aminophenol,
1,4-Phenylendiamine,
N, N-Dimethyl-4-aminothiophenol,
N, N'-Bis-(1,4-dimethylpentyl)-1,4-phenylendiamin
gewählt ist.

9. Zusammensetzung nach Anspruch 1, worin die organische Säure eine C6-C22-Carbonsäure bedeutet.

10. Zusammensetzung nach Anspruch 1, worin die organische Säure C₆- bis C₁₈-Carbonsäure bedeutet.

11. Zusammensetzung nach Anspruch 1, worin die Nitroxylverbindung aus:
1-Oxyl-2,2,6,6-tetramethylpiperidin,
1-Oxyl-2,2,6,6,-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6,-tetramethylpiperidin-4-yl-stearat,
1-Oxyl-2,2,6,6,-tetramethylpiperidin-4-yl-benzonat,
1-Oxyl-2,2,6,6-tetramethylpiperidin-4-yl-4-tert.-butylbenzoat,
Bis(1-oxyl-2,2,6,6-tetramethylpiperidin-4-yl)succinat,
Bis(1-oxyl-2,2,6,6-tetramethylpiperidin-4-yl)sebacat,
Bis(1-oxyl-2,2,6,6-tetramethylpiperidin-4-yl)sebacat,
Bis(1-oxyl-2,2,6,6-tetramethylpiperidin-4-yl)adipate,
Bis(1-oxyl-2,2,6,6-tetramethylpiperidin-4-yl)n-butylmalonat,
Bis(1-oxyl-2,2,6,6-tetramethylpiperidin-4-yl)phthalat,
Bis( 1-oxyl-2,2,6,6-tetramethylpiperidin-4-yl)isophthalat,
Bis(1-oxyl-2,2,6,6-tetramethylpiperidin-4-yl)terephthalat,
Bis( 1-oxyl-2,2,6,6-tetramethylpiperidin-4-yl)hexahydoterephthalat,
N,N'-Bis(1-oxyl-2,2,6,6-tetramethylpiperidin-4-yl)adipamin,
N-(1-Oxyl-2,2,6,6-tetramethylpiperidin-4-yl)caprolactam,
N-(1-Oxyl-2,2,6,6-tetramethlypiperidin-4-yl)dodecylsuccinimid,
2,4,6-Tris-[N-butyl-N-(1-oxyl-2,2,6,6-tetramethylpiperidin-4-yl]-s-triazin und
4,4'-Ethylenbis(1-oxyl-2,2,6,6-tetramethlypiperidin-3-on)
4-Acetylamino-2,2,6,6-tetramethlypiperidin-N-oxyl
gewählt ist.

12. Zusammensetzung nach Anspruch 1, worin das Nitroxyl 1-Oxyl-2,2,6,6-tetramethylpiperidin-4-ol oder Bis(1-oxyl-2,2,6,6,-tetramethylpiperidin-4-yl) sebacat bedeutet.

13. Verfahren zur Inhibierung der vorzeitigen Polymerisation einer vinylaromatischen oder aliphatischen Verbindung während der Destillation, Reinigung oder Lagerung, mit der Stufe, bei der eine effektiv inhibierende Menge, die ausreicht, um die vorzeitige Polymerisation während der Destillation, Reinigung oder Lagerung einer Mischung aus
i) 1 bis 99 Gew.-%, bezogen auf das Gesamtgewicht der Bestandteile (i) und (ii), einer Mischung aus mindestens einem aromatischen Amin und mindestens einer organischen Säure in einem Molverhältnis von 10:1 bis 1:10 und
ii) 99 bis 1 Gew.-%, bezogen auf das Gesamtgewicht der Bestandteile (i) und (ii), mindestens einer stabilen radikalischen Verbindung, zu verhindern,
worin dieses aromatische Amin, diese organische Säure und diese radikalische Verbindung die in Anspruch 1 definierten Bedeutungen aufweisen.

14. Verfahren nach Anspruch 13, worin die Mischung so gewählt wird, dass sie eine Zusammensetzung nach einem der Ansprüche 1 bis 12 bildet.

15. Verfahren nach Anspruch 13 oder 14.

## Revendications

1. Composition monomère, stabilisée contre la polymérisation prématurée, comprenant :
a) un monomère non saturé éthyléniquement ou un mélange de monomères polymérisables par initiation de radicaux libres, et
b) une quantité efficace, suffisante pour inhiber la polymérisation prématurée du composant (a) d'un mélange de :
i) 1 à 99 % en poids, sur la base du poids total des composants (i) et (ii) d'un mélange d'au moins un amine aromatique et au moins un acide organique dans un rapport molaire de 10:1 à 1:10, et
ii) 99 à 1 % en poids sur la base du poids total des composants (i) et (ii) d'au moins un composé à radicaux stables,
où l'amine aromatique a la formule (1) 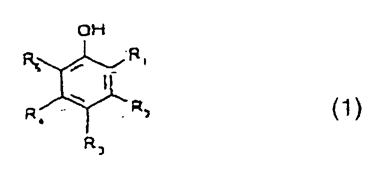 (où Q est 0 ou S ou N-Z
et où au moins un R est un alkylamine de la structure (2) 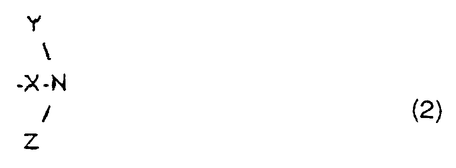 où Y, Z sont identiques ou différents et comprennent un alkyl ou hydrogène à chaîne ramifiée ou droite C₁ à C₉ et où X est soit une liaison covalente soit un alcène C₁ à C₄, et où chaque R restant est indépendamment benzyl, alkyl ou hydrogène à chaîne ramifiée ou droite C₁ à C₄ avec la possibilité que deux R ou plus peuvent être reliés pour former un ou plusieurs anneaux) ;
où l'acide organique est un acide carboxylique ; et
où le composé à radicaux stables est un nitroxyl stériquement retardé de la formule 3 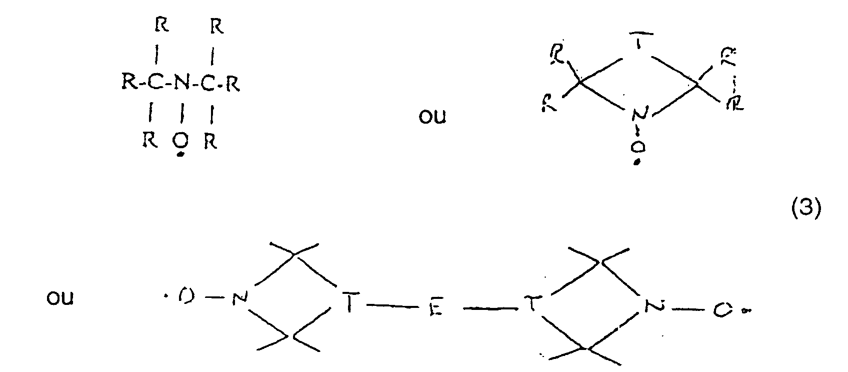 (où R est un hydrogène, alkyl ou aryl et T est un groupe nécessaire pour former un anneau et où deux groupes T ou plus peuvent être liés par un groupe de liaison E).

2. Composition selon la revendication 1, **caractérisée en ce que** le poids du composant (i) est 50 à 99 % et le poids du composant (ii) est 50 à 1 %.

3. Composition selon la revendication 2, **caractérisée en ce que** le poids du composant (i) est 67 à 99 % et le poids du composant (ii) est 33 à 1 %.

4. Composition selon l'une des précédentes revendications, **caractérisée en ce que** le rapport molaire amine aromatique/acide organique est 3:1 à 1:3.

5. Composition selon la revendication 1, **caractérisée en ce que** Q est de l'oxygène.

6. Composition selon la revendication 1 ou 5 **caractérisée en ce que** X est du méthylène.

7. Composition selon l'une des revendications 1,-5 ou 6, **caractérisée en ce que** Y et Z sont du méthyle.

8. Composition selon l'une des précédentes revendications, **caractérisée en ce que** l'amine est choisi à partir de
N,N-diméthyle-1,4-phénylène-diamine
3,5-butylditertiaire-4-oxy, N,N-diméthylebenzylamine
3,5-butylditertiaire-4-oxy-N ,N-diéthylebenzylamine
N,N-diméthyl-2,6-butylditertiaire-4-amino-phénol
1,4-phénylène-diamine
N,N-diméthyle-4-aminothiophénol
N,N'-bis-(1,4 diméthylepentyl)-1,4-phénylènediamine.

9. Composition selon la revendication 1, **caractérisée en ce que** l'acide organique est un acide carboxylique C₆ à C₂₂.

10. Composition selon la revendication 1, **caractérisée en ce que** l'acide organique est un acide carboxylique C₆ à C₁₈.

11. Composition selon la revendication 1, **caractérisée en ce que** le composé nitroxyl est choisi à partir de :
1-oxyl-2,2, 6,6-tétraméthylpiperidine,
1-oxyl-2,2,6,6-tétraméthylpiperidine-4-ol,
1-oxyl-2,2,6,6-tétraméthylpiperidine-4-un,
1-oxyl-2,2,6,6-tétraméthylpiperidine-4-yl acétate,
1,oxyl-2,2,6,6-tétraméthylpiperidine-4-yl 2-éthylhexanoate,
1-oxyl-2,2,6,6-tétraméthylpiperidine-4-yl stéarate,
1-oxyl-2,2,6,6-tétraméthylpiperidine-4-yl benzoate,
1-oxyl-2,2,6,6-tétraméthylpiperidine-4-yl 4-tert-butylbenzoate,
bis (1-oxyl-2,2,6,6-tétraméthylpiperidine-4-yl) succinate,
bis (1-oxyl-2,2,6,6-tétraméthylpiperidine-4-yl) sébacate,
bis (1-oxyl-2,2,6,6-tétraméthylpiperidine-4-yl) sébacate,
bis (1-oxyl-2,2,6,6-tétraméthylpiperidine-4-yl) adipate,
bis (1-oxyl-2,2,6,6-tétraméthylpiperidine-4-yl) n-butylmalonate,
bis (1-oxyl-2,2,6,6-tétraméthylpiperidine-4-yl) phtalate,
bis (1-oxyl-2,2,6,6-tétraméthylpiperidine-4-yl) isophtalate,
bis (1-oxyl-2,2,6,6-tétraméthylpiperidine-4-yl) téréphtalate,
bis (1-oxyl-2,2,6,6-tétraméthylpiperidine-4-yl) hexahydotéréphtalate,
N,N'bis (1-oxyl-2,2,6,6-tétraméthylpiperidine-4-yl) adipamine,
N-(1-oxyl-2,2,6,6-tétraméthylpiperidine-4-yl) caprolactame,
N- (1-oxyl-2,2,6,6-tétraméthylpiperidine-4-yl) dodécylsuccinimide,
2,4,6-tris-(N-butyl-N-(1-oxyl-2,2,6,6-tétraméthylpiperidine-4-yl)-s-triazine, et
4,4'-éthylènebis(1-oxyl-2,2,6,6-tétraméthylpiperidine-3-un)
4-acétylamino 2,2,6,6-tétraméthylpiperidine-N-oxyl.

12. Composition selon la revendication 1, **caractérisée en ce que** le nitorxyl est 1-oxyl-2,2,6,6-tétraméthylpipedine-4-ol, ou bis(1-oxyl-2,2,6,6-tétraméthylpiperidine-4-yl) sébacate.

13. Procédé pour inhiber la polymérisation prématurée d'un composé aromatique ou aliphatique vinyle pendant la distillation, la purification ou le stockage, comprenant l'étape d'incorporation d'une quantité inhibante efficace, suffisante pour empêcher la polymérisation prématurée pendant la distillation, la purification ou le stockage d'un mélange de :
i) 1 à 99 % en poids, sur la base du poids total de composants (i) et (ii), d'une addition d'au moins un amine aromatique et au moins un acide organique dans un rapport molaire de 10:1 à 1:10 et
ii) 99 à 1 % en poids, sur la base du poids total des composés (i) et (ii) d'au moins un composé à radicaux stables,
**caractérisé en ce que** ledit amine aromatique, ledit acide organique et ledit composé radical sont selon définition à la revendication 1.

14. Procédé selon la revendication 13, **caractérisé en ce que** ledit mélange est choisi pour assurer une composition conforme aux revendications 1 à 12.

15. Procédé selon la revendication 13 ou 14.
